# EUROPEAN PATENT SPECIFICATION

(11) **EP 3 381 278 B1**
(45) Date of publication and mention of the grant of the patent: **13.07.2022**
(21) Application number: 18164988.0
(22) Date of filing: 29.03.2018
(51) Int. Cl.: A01K 67/027, C12N 15/873, C12N 15/113

(54) **METHOD FOR PREPARING A CANINE MODEL OF ATHEROSCLEROSIS**
VERFAHREN ZUR HERSTELLUNG EINES KYNOLOGISCHEN MODELLS DER ATHEROSKLEROSE
PROCÉDÉ DE PRÉPARATION D'UN MODÈLE CANINE D'ATHÉROSCLÉROSE

(30) Priority: 29.03.2017 CN 201710197156
(43) Date of publication of application: 03.10.2018
(73) Proprietor: Beijing Sinogene Biotechnology Co., Ltd., 102200, Beijing (CN)
(72) Inventor: ZHENG, Min, Beijing (CN); ZHAO, Jianping, Beijing (CN); MI, Jidong, Beijing (CN)
(74) Representative: Sommer, Andrea

(56) References cited:
- DONGSHAN YANG ET AL: "Effective gene targeting in rabbits using RNA-guided Cas9 nucleases", JOURNAL OF MOLECULAR CELL BIOLOGY, vol. 6, no. 1, February 2014 (2014-02), pages 97-99, XP055495958, ISSN: 1674-2788, DOI: 10.1093/jmcb/mjt047
- JI DIANA ET AL: "Efficient creation of an APOE knockout rabbit", TRANSGENIC RESEARCH, SPRINGER NETHERLANDS, NL, vol. 24, no. 2, 13 September 2014 (2014-09-13), pages 227-235, XP035467340, ISSN: 0962-8819, DOI: 10.1007/S11248-014-9834-8 [retrieved on 2014-09-13]
- QINGJIAN ZOU ET AL: "Generation of gene-target dogs using CRISPR/Cas9 system", JOURNAL OF MOLECULAR CELL BIOLOGY, vol. 7, no. 6, 12 October 2015 (2015-10-12), pages 580-583, XP055495917, ISSN: 1674-2788, DOI: 10.1093/jmcb/mjv061
- WU YIN ET AL: "Plasma lipid profiling across species for the identification of optimal animal models of human dyslipidemia", JOURNAL OF LIPID RESEARCH, vol. 53, no. 1, 2012, pages 51-65, XP055495920, US ISSN: 0022-2275, DOI: 10.1194/jlr.M019927
- LINDBLAD-TOH KERSTIN ET AL: "Genome sequence, comparative analysis and haplotype structure of the domestic dog", NATURE, MACMILLAN JOURNALS LTD, LONDON, GB, vol. 438, no. 7069, 8 December 2005 (2005-12-08), pages 803-819, XP002429578, ISSN: 0028-0836, DOI: 10.1038/NATURE04338
- Anonymous: "Canis lupus familiaris Annotation Report", , 17 September 2015 (2015-09-17), XP055495945, Retrieved from the Internet: URL:https://www.ncbi.nlm.nih.gov/genome/an notation_euk/Canis_lupus_familiaris/104/ [retrieved on 2018-07-30]
- Anonymous: "Ensembl genome browser 67: Canis lupus familiaris - Gene summary - Gene: APOE_CANFA (ENSCAFG00000004617)", , May 2012 (2012-05), XP055495953, Retrieved from the Internet: URL:http://may2012.archive.ensembl.org/Can is_familiaris/Gene/Summary?g=ENSCAFG000000 04617;r=1:113416152-113418736;t=ENSCAFT000 00007432 [retrieved on 2018-07-30]

## Description

### FIELD OF THE INVENTION

The present invention relates to a method for establishing an APOE gene knock-out canine model demonstrating an atherosclerotic phenotype with the use of gene knock-out technology.

### BACKGROUND OF THE INVENTION

Atherosclerosis (AS) is an elderly frequently occurring disease caused by multiple factors such as inheritance and environment. It is a main cause of cardiovascular diseases such as coronary heart disease, cerebral infarction, peripheral vascular disease, etc. If patients of coronary atherosclerosis are in pipe diameter stenosis of above 75%, angina pectoris, myocardial infarction, arrhythmia, and even sudden death may occur. Cerebral atherosclerosis may cause cerebral ischemia and encephalatrophy, or lead to hemorrhage due to cerebral vascular rupture.

The etiology of AS is complicated. The incidence of AS is associated with a variety of pathogenic factors, and often occurs in large and medium-sized elastic vessels, and endometrium and underneath the endometrium of muscular arterial wall. AS is characterized by lipid deposition and endometrial thickening, and forms atherosclerosis lesions or fibrous lipid plaques. Among the multiple pathogenic factors, lipid metabolism disorder, especially hypercholesterolemia is most closely related. Apolipoprotein (Apo) E is an important component of plasma lipoproteins. It serves an important role in adjusting plasma cholesterol level and transportation and metabolism of lipid, and is an important molecular target in occurrence and development of hyperlipemia and AS, etc. Therefore, ApoE is the key in the occurrence and development of AS.

In recent years, the incidence of AS showed younger and rising trends. It is therefore necessary to establish atherosclerosis animal models to investigate thoroughly the etiology and the medicines. Rats and mice are the most popularly used animal models, while rats are AS resistant. Further, although the formed pathologic change thereof is similar to human's early lesion, it is difficult to form later lesion similar to that of human body, and it is not convenient to take blood from mice.

Canine is one of the commonly used laboratory animals in fundamental medical research and teaching, and especially, plays an important role in experimental researches such as physiology, pharmacology and pathophysiology. Canine also has hereditary diseases comparatively similar to that of human. Canine has less hereditary diseases, good experimental repeatability, well-developed blood circulation and nervous system. Canine is similar in digestive system and internal organs, and is closer in toxicological reactions to that of human. Therefore, canine is especially suitable for investigations in pharmacology, circulatory physiology, ophthalmology, toxicology, and surgery, etc. Further, canines are gentle and are easy to tune, and can cooperate well in experimental research through short-term training. Therefore, canines are regarded to be comparatively ideal for experimental research in international medical and biological fields.

Currently used methods for establishing a canine disease model mainly include the methods, such as feeding method, mechanical damage method, immunization method, etc. Feeding method, mechanical damage method and immunization method are to use special ways to induce healthy animals to have disease phenotypes. These methods have problems that the inductive canine animal models do not show disease phenotype, the duration of phenotype thereof is short, and the inductive canine animal models cannot simulate human disease symptoms.

The shortcomings of the above inductive canine animal models can be overcome by using gene engineering for gene knock-out or transgenic modification in non-human animals to establish disease animal models. However, the most well established experimental animals for the application of gene knock-out or transgenic modification technology are mice, and the application to large mammal animal models is still under exploration. Even there are some reports on the establishment of gene knock-out animal models of large mammal animals such as bovine, sheep, pig, monkey, etc, it is a great difficulty for in vitro manipulation of canine oocytes and the embryo, and thus the difficulties for establishing gene knockout or genetically modified model canines are increased due to the big difference of the breeding physiology between canines and other mammals. Thus, even there is a great amount of demand for diseases model canines having gene knock-out or transgenic modification, there are few reports on the successful establishment of diseases model canines having gene knock-out or transgenic modification worldwide. There is no report on model canines having atherosclerosis gene knock-out or transgenic modification at all.

Therefore, it is highly desirable to establish atherosclerosis gene knock-out or transgenic modification model canines, whose disease symptoms are primary symptoms. The disease phenotype thereof can last a long period of time, and the disease is hereditary, and consequently, offsprings of disease model canines can be obtained through natural reproduction. Thus, suitable experimental animal models having gene knock-out or transgenic modification can be provided for the study of cardiovascular diseases, and the research and development of related medicines.

### SUMMARY OF THE INVENTION

In one aspect, the present invention provides a method for establishing an APOE gene knock-out model canine, comprising the following steps: (1) determining a targeting site sequence directed to an exon sequence of canine APOE gene sequence; (2) synthesizing sgRNA sequence and the complementary sequence thereof according to the targeting site sequence determined in step (1), then linking the synthesized sequence with a skeleton vector to construct a sgRNA targeting vector; (3) in vitro transcripting the sgRNA targeting vector to obtain mRNA thereof, and in vitro transcripting CRISPR/Cas9 to obtain mRNA thereof; (4) mixing the mRNA of sgRNA and mRNA of CRISPR/Cas9 obtained in step (3), and then intracytoplasmic injecting thereof into the fertilized ovum; and (5) transplanting the fertilized ovum into less bleeding fallopian tubes of a female canine of which both fallopian tubes have been embryo flushed.

The targeting site sequence is determined directed to sequence of exon 3 (SEQ ID NO: 2),
and, the targeting site sequence in step (1) is determined as follows: 5'-CCGGGTGGCAGACTGGCCAGCCC-3' (SEQ ID NO: 4),
and the sgRNA sequence and the complementary sequence thereof synthesized in step (2) are as follows:
   sgRNA sequence: ataGGGCTGGCCAGTCTGCCACCgt (SEQ ID NO: 5); and
   the complementary sequence of sgRNA sequence: taaaacGGTGG CAGACTGGCCAGCC (SEQ ID NO: 6).

Preferably, the skeleton vector is T7-gRNA commercially available from Addgene.

In the second aspect, in step (4) of the above second aspect, mRNA of sgRNA and mRNA of CRISPR/Cas9 obtained in step (3) are mixed, and subsequently is intracytoplasmic injected into a somatic cell, and then the somatic cell nuclear is transplanted into enucleated oocytes; and in step (5), the enucleated oocytes are transplanted into one of fallopian tubes of a female canine of which both fallopian tubes have been embryo flushed.

In the third aspect, the present invention provides a canine APOE gene targeting vector, which consists of sgRNA sequence and the complementary sequence thereof designed directed to the targeting site sequence in the exons of canine APOE gene, and the skeleton vector.

The exon is exon 3 (SEQ ID NO: 2) of the canine APOE gene. Preferably, the skeleton vector is T7-gRNA commercially available from Addgene.

Preferably, the targeting site sequence is as follows:
5'-CCGGGTGGCAGACTGGCCAGCCC-3' (SEQ ID NO: 4).

Preferably, the sgRNA sequence and the complementary sequence thereof are as follows:
sgRNA sequence: ataGGGCTGGCCAGTCTGCCACCgt (SEQ ID NO: 5); and
the complementary sequence of sgRNA sequence: taaaacGGTGG CAGACTGGCCAGCC (SEQ ID NO: 6).

In the fourth aspect, the present invention provides a somatic cell, tissue or organ of APOE gene knock-out canine obtained through the method of any one of the above first to the third aspects.

Preferably, the somatic cell comprises a sequence shown by cctggaccagggaggct (SEQ ID NO: 7).

Preferably, the somatic cell is ear fibroblast BGD-APOEKO-EFO of APOE gene knock-out beagle canine, which is deposited in China General Microbiological Culture Collection Center (CGMCC) on Mar 1st, 2017 with a CGMCC deposit No. 13804.

In the fifth aspect, the present invention provides a primer pair for detecting APOE gene knock-out canine comprising a genomic sequence comprising a sequence fragment as shown by cctggaccagggaggct (SEQ ID NO: 7), characterized in that the primer pair is designed directed to the sequence as shown by cctggaccagggaggct (SEQ ID NO: 7).

Preferably, the sequences of the primer pair are as follows:
Forward primer F: 5'-CATTGTTGTCAGGCAGGTAGC-3' (SEQ ID NO: 8); and
Reverse primer R: 5'- GAAGGGTGCGAGGGATTGA-3' (SEQ ID NO: 9).

In the sixth aspect, the present invention provides a kit for detecting APOE gene knock-out canine comprising a genomic sequence comprising a sequence fragment as shown by cctggaccagggaggct (SEQ ID NO: 7), characterized in that the kit comprises a primer pair designed directed to the sequence as shown by cctggaccagggaggct (SEQ ID NO: 7).

Preferably, the sequences of the primer pair are as follows:
Forward primer F: 5'-CATTGTTGTCAGGCAGGTAGC-3' (SEQ ID NO: 8); and
Reverse primer R: 5'-GAAGGGTGCGAGGGATTGA-3' (SEQ ID NO: 9).

Canine APOE gene comprises totally four exons, and the translation initiation site is located at the second exon. The present invention carries out gene targeting at the third exon, resulting in frameshift mutation of the genomic sequence thereof, and the translation is terminated at the 63^{rd} amino acid. The APOE protein cannot be fully expressed, and thus the purpose of gene knock-out is achieved. Besides this site, gene targeting is carried out at any sequence of Exon 2, Exon 3 and Exon 4 of the canine APOE gene, which causes changes of gene sequence and leads to advanced termination of amino acid translation. APOE protein can't be fully expressed. An incompletely expressed APOE protein cannot perform the original functions, and thus the purpose of canine APOE gene knock-out can also be achieved.

The present invention prepares an APOE gene knock-out canine through choosing a targeting site sequence based on exons of the canine APOE gene sequence, constructing sgRNA targeting vector and CRISPR/Cas9 expression vector according to the targeting site sequence using gene editing, then intraplasmic injecting the mRNA of sgRNA and mRNA of CRISPR/Cas9 obtained through in vitro transcription into a canine fertilized ovum, and transplanting the canine fertilized ovum into one of fallopian tubes of a female canine of which both fallopian tubes have been embryo flushed. This is the first time to obtain APOE gene knock-out canine successfully in the world. And both fallopian tubes have been embryo flushed prior to transplanting the fertilized ovum into one of the two fallopian tubes, which increases numbers of transgenic fertilized ovum, and avoids influence of embryos in the fallopian tube that was not embryo flushed on the implantation of the gene knock-out embryos, compared to embryo flushing only one side of the fallopian tube. Thus, the utilization efficiency of the fertilized ovum and the survival rate of the transgenic canine are significantly increased.

In addition, the APOE gene knock-out canines obtained in the present invention will provide disease animal models of tremendous application value for medical research, and lay foundations for pushing studying of cardiovascular disease and screening of cardiovascular disease drugs.

Abbreviations and key terms definitions:
APOE: an apolipoprotein E, which is one of the apolipoproteins synthesized mainly in liver and brain tissue, and is a constituent of nervous system and plasma lipoproteins. APOE participates in metabolism process of cholesterol and triglyceride in blood by bonding low-density lipoprotein receptor to take in low-density lipoprotein. Human APOE gene is located at the long arm of the 19th pair of chromosome and has a length of 37kb. This gene comprises four exons and three introns. The cDNA thereof has a length of 1.63kb, and the initiating product thereof is a protein comprising 317 amino acids. After being cleaved by a signal peptide comprising 18 amino acids, it becomes a mature protein consisting of 299 amino acids. Canine gene of APOE is located at number 1 chromosome of canine, and this gene has a total length of 2788bp, which has totally four exons, and six CDS regions for encoding proteins, which encode 323 amino acids.
ICI: intracytoplasmic injecting, which refers to injecting gene into cytoplasm of fertilized ovum through micromanipulation with use of a microinjection needle.
AS: atherosclerosis. Lipid metabolism disorder is the lesion foundation of atherosclerosis. The disease is characterized in that lesion of the involved arterial starts from endometrium. Accumulation of lipid and complex carbohydrate occurs at first in general, followed by bleeding and thrombosis. Hereafter, proliferation and calcinosis of fibrous tissue are further developed, and gradual metamorphosis and calcification of arterial media happen, which lead to thickening and hardening of arterial wall and vascular stenosis. The lesion often involves large and medium muscular artery. Once the lesion is developed enough to block the artery cavity, the tissue or organ supplied by the arteries will be ischemic or necrotic. Since the lipid accumulated in the arterial intimas is yellow atherosclerosis, it is called atherosclerosis.

### BRIEF DESCRIPTION OF THE DRAWINGS

FIG. 1 is a schematic diagram of canine APOE gene targeting site sequence.
FIG. 2 shows Exon 3 sequence of the APOE gene of a wild type canine and mutated sequence of Exon 3 sequence of the APOE gene of the gene knock-out canine numbered 161207.
FIG. 3 shows sequence comparison of APOE gene from different origins.
FIG. 4 shows a sequencing peak diagram of the APOE gene edited canine.

### DETAILED DESCRIPTION OF THE INVENTION

Technical solutions of the present invention are further described in below through combining examples and drawings of the Description. These examples are for illustrating rather than setting limit to the scopes of protection of the present invention.

### Embodiment 1: Constructing, in vitro transcription and verification of transgenic targeting vector

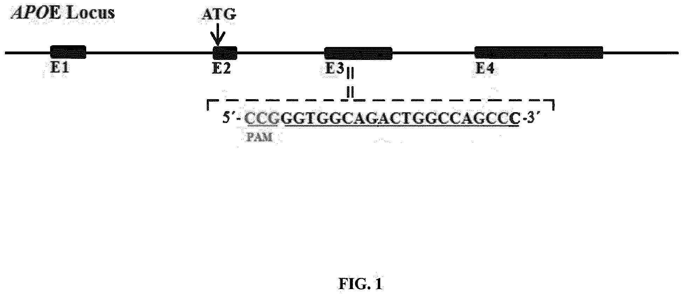

Choosing a targeting site sequence: 5'-CCGGGTGGCAGACTGGCCAGCCC-3' (SEQ ID NO: 4) (see FIG. 1) based on Exon 3 of canine APOE gene according to canine APOE gene sequence information provided by NCBI. The sgRNA sequence identifying the targeting site sequence is 5'-GGGCTGGCCAGTCTGCCACC-3' (SEQ ID NO: 10). When constructing a vector, the skeleton vector T7-gRNA (commercially available from Addgene) is enzyme digested with BbsI for subsequent experiments; sgRNA sequence: ataGGGCTGGCCAGTCTGC CACCgt (SEQ ID NO: 5) and sgRNA complementary sequence: taaaacGGTGGCAGACTGGCCAGCC (SEQ ID NO: 6) are designed; sgRNA sequence and the sgRNA complementary sequence are annealing linked, and then linked with enzyme digested T7-gRNA plasmid. A PCR product is recovered after PCR amplifying the T7-sgRNA plasmid, and an in vitro transcription kit is used for in vitro transcriptipn the PCR product of T7-sgRNA.

At first, the plasmid of CRISPR/Cas9 is linearized. The reacting system is as follows: 30µg plasmid, 5µl restriction endonuclease AflII; 10µl of 10×Buffer and ddH₂O, the total volume is 100µl. Then 100µl of phenol: chloroform: isopropyl alcohol (25:24:1) is added to purify the linearied plasmid DNA, and then 12,000g centrifugation for 5min; sucking 50µl supernatant into a 1.5ml centrifugation tube without Rnase, adding sodium acetate in 1/10 volume and anhydrous ethanol in 3 times volume to precipitate plasmid DNA, and then 12,000g centrifugation for 5min; discarding the supernatant, discard the remaining supernatant at the best; adding 150µl ethanol of 70% to wash the plasmid, and then 12,000g centrifugation for 5min; drying in air for 3-5min, dissolving DNA with 15µl ddH₂O of RNase-free, and measuring the concentration.

In vitro transcription mRNA with a kit (Ambion):
The system of in vitro transcription is as follows: 1µg linearized plasmid DNA, 10µl 2×NTP/CAP, 2µl 10×Buffer, 2µl RNA synthetase and ddH₂O, the total volume 20µl. After mixing homogeneously, incubating for 1hr at 37°C; adding 1µl of TURBO DNA enzyme, digesting plasmid template, and incubating for 30min at 37°C. Then, mixing 20µl in vitro transcription product, 20µl 10×Reaction Buffer, 10µl ATP (10mM), 2.5µl RNase inhibitor, 2µl Poly (A) polymerase and nuclease-free ddH₂O to formulate a system of in vitro transcription mRNA plus poly (A), total volume of 100µl, and incubating for 1hr at 37°C. After incubation, adding 350µl binding buffer to the reaction system and mixing homogeneously through blowing; then adding 250µl anhydrous ethanol, and mixing homogeneously; then transferring the sample into an mRNA purification column, then 10,000g centrifugation for 1min at room temperature; discarding the filtrate, and then reloading the column, rinsing the column with 500µl eluent, and then 10,000g centrifugation for 1min at room temperature; repeating the rinsing one time, discarding the filtrate, centrifugation of the empty column for 1min to rinse off impurities such as proteins; then the column is placed into a new centrifugation tube, adding 50µl RNA eluent to the central position of the column, covering the lid and incubating for 10min at 65°C, then 10,000g centrifugation for 1min at room temperature; and measuring quality and concentration of the RNA.

The sgRNA of CRISPR and mRNA of Cas9 are mixed so that sgRNA has a final concentration of 20ng/µl, and Cas9 has a final concentration of 200ng/µl, storing at -80°C for cytoplasmic injection.

The constructed sgRNA and Cas9 plasmid are co-transferred to canine skin fibroblasts, and G418 is used for screening. DNA is extracted from cell clones obtained by screening as a template, and the following primer pair is used to conduct PCR, which amplifies a DNA fragment of total 660 bp at the upstream and downstream of the target being identified and cleaved by sgRNA:
Forward primer F: 5'-CATTGTTGTCAGGCAGGTAGC-3' (SEQ ID NO: 8);
Reverse primer R: 5'- GAAGGGTGCGAGGGATTGA-3' (SEQ ID NO: 9).

The targeting fragment obtained through PCR amplifying is subjected to DNA sequencing to determine the targeting efficiency of the vector. Total of thirty (30) cell clones are obtained after transfection and screening, wherein twenty-six (26) cell clones have the gene mutation in the region of the targeting site showed by the PCR sequencing. The mutation efficiency is 86.7%, which proves that the constructed vector has a high accuracy, and the targeting efficiency is higher. Therefore, the constructed vector can be used for the preparation of APOE gene knock-out canine.

### Embodiment 2: Embryo transplanting of APOE gene knock-out canine

Total of 13 female beagle canines of natural estrous are used as donors of fertilized ova and also receptors of embryo transplanting for experimental research. Blood sample is taken to measure progesterone level in serum. If the progesterone concentration is at 4-7ng/ml, the ovulatory period is determined. Natural mating is performed after 48hr of ovulation, and followed by flushing the fertilized ova. 65 fertilized ova are collected from 13 female canines. After the fertilized ova are collected, they are subjected to removing cumulus granulosa cells by using TCM199 medium comprising 0.1% hyaluronidase, followed by putting into droplets of HEPES buffered TCM199 medium (HM, GIBCO11150), and then placing on an inverted microscope equipped with a micromanipulator. A mixture comprising mRNA of sgRNA and mRNA of Cas9 prepared in Embodiment 1 at a ratio of 1:1 in volume is sucked with a microinjection needle, and then injected into cytoplasm of a fertilized ovum. The fallopian tube is flushed with 10 mL of HEPES buffered TCM199 medium (HM, GIBCO11150) comprising 10% fetal bovine serum, and the ovum flushing fluid is discharged from the injection needle ligated at umbrella of the fallopian tube, and is collected into a 10ml centrifugation tube.

After the intraplasmic injection, the embryos are loaded into an embryo transplanting tube, and the embryos in the embryo transplanting tube are injected from the fallopian tube umbrella into the less bleeding one of fallopian tubes when embryo flushing.

**Table 1: Embryo Transplanting Results**

| No. of receptor canines | Number of transplanted fertilized ova | Number of offsprings | Numbers of gene knock-out canine |
|---|---|---|---|
| FRA1115 | 8 | 0 | 0 |
| FRA1121 | 4 | 0 | 0 |
| FRA1126 | 5 | 0 | 0 |
| FRA1118 | 1 | 0 | 0 |
| FRA1124 | 5 | 4 | 1 |
| FRA1123 | 6 | 1 | 0 |
| FRA1129 | 7 | 1 | 0 |
| FRA1024 | 8 | 0 | 0 |
| FRA1130 | 6 | 2 | 0 |
| FRA1139 | 2 | 1 | 0 |
| FRA1137 | 2 | 6 | 1 |
| FRA1140 | 8 | 0 | 0 |
| FRA1146 | 3 | 1 | 0 |
| Total | 65 | 13 | 2 |

It can be seen from the above Table 1 that 13 female beagle canines transplanted with 65 fertilized ova bring total of 13 offsprings, and two gene knock-out canines are obtained. The detection and verification are given in the following examples.

### Embodment 3: Gene mutation detection of APOE gene knock-out canine

After the puppies are born, ear tissue and tail tissue are collected for identification. After the tissue block is fragmented in a centrifugation tube, protease K is added for water bath and cleavage at 56°C for 1~3h. Then 700µl of Genomic Lysis Buffer sucked with a pipette is added to the cleavage system, mixing homogeneously through turning upside down, and then 10000g centrifugation for 1min. The supernatant was sucked to a purifying column with a pipette, 10000g, standing for 1min at room temperature. A new collection tube was replaced, and 200µl DNA Pre-Wash Buffer was added to the centrifugation column, 10000g, followed by standing for 1min at room temperature, centrifuging for 1min, and discarding wasted liquid. 400µl g-DNA Wash Buffer was added to the centrifugation column, 10000g, standing for 1min at room temperature, centrifuging for 1min, and discarding the wasted liquid. The purifying column and collecting tube are re-centrifugated, 10000g, centrifuging for 2min. The purifying column was placed in a newly replaced 1.5ml centrifugation tube, 50µl Elution Buffer was added to elute DNA, followed by standing for 2min at room temperature, and then 12000rpm, centrifuging for 1min. The obtained solution is canine genomic DNA.

The canine genomic DNA was used as a template to carry out PCR, the primers are as follows:
Forward primer F: 5'-CATTGTTGTCAGGCAGGTAGC-3' (SEQ ID NO: 8);
Reverse primer R: 5'- GAAGGGTGCGAGGGATTGA-3' (SEQ ID NO: 9),

After amplification, a DNA fragment of total 660bp at the upstream and downstream of the target being identified and cleaved by sgRNA. The targeting fragment obtained through PCR amplifying is undergone DNA sequencing, and is compared with canine APOE gene sequence provided by NCBI database to determine the mutation type of the APOE gene.

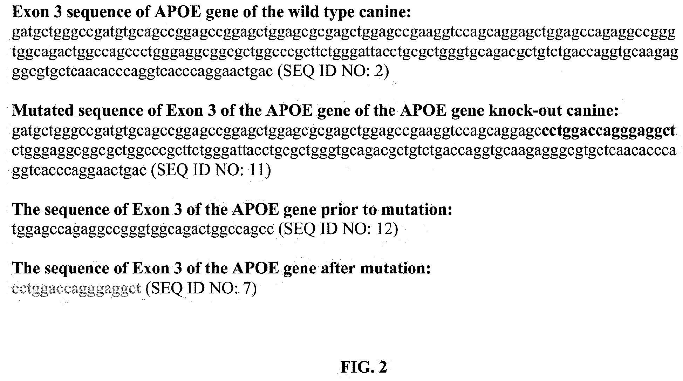

Upon sequencing and sequence alignment, it is demonstrated that the mutation is occurred in Exon 3 targeting site of APOE gene of one male and one female (total of 2 puppies) among the thirteen puppies, wherein the mutation of the male canine (numbered 161207) is that a fragment of 34 bp was deleted and a fragment of 17bp was inserted at the same time, which is APOE gene homozygous double knock-out, resulting in the APOE protein starting mutation from the 37^{th} amino acid, and terminated the translation at the 63^{th} amino acid; the other female canine (numbered 170111) has a heterozygous mutation of deleting 33bp at one side and deleting 51bp at the other side. FIG. 2 shows Exon 3 sequence (SEQ ID NO: 2) of APOE gene of a wild type canine, and mutated sequence (SEQ ID NO: 11) of Exon 3 sequence of APOE gene of gene knock-out canine numbered 161207, which indicates that a fragment of 34bp was deleted, and a fragment of 17bp was inserted at the same time, and the bold part of Exon 3 sequence of APOE gene of the gene knock-out canine (numbered 161207) denotes the added fragment of 17bp. Particularly, the sequence of the corresponding site prior to mutation is tggagccagaggccgggtggcagactggccagcc (SEQ ID NO: 12), and the sequence after mutation is cctggaccagggaggct (SEQ ID NO: 7).

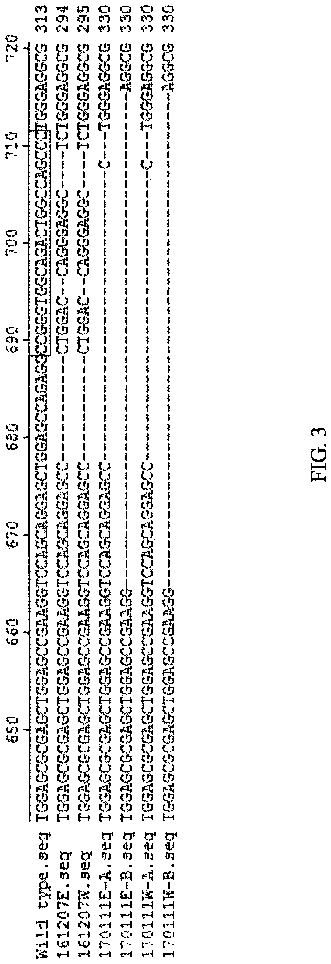

FIG. 3 shows the sequences alignment among the sequence of the 641^{st} to the 720^{th} nucleotide of Exon 3 of APOE gene of a wild type canine, the corresponding sequences of Exon 3 of APOE gene from ear and tail tissues of APOE gene knock-out canine (numbered 161207) respectively, and the corresponding sequences of Exon 3 of APOE gene from ear and tail tissues of APOE gene knock-out canine (numbered 170111) respectively. Note that the targeting sequence is in the box; letter suffix E after the numeric number indicates ear tissue, suffix W after the numeric number indicates tail tissue; A and B indicate the number of alleles of APOE heterozygous knock-out canine numbered 170111.

The Ear fibroblast BGD-APOEKO-EFO of APOE gene knock-out beagle canine numbered 161207 is deposited in China General Microbiological Culture Collection Center (CGMCC) on March 1, 2017, with a CGMCC deposit No. 13804.

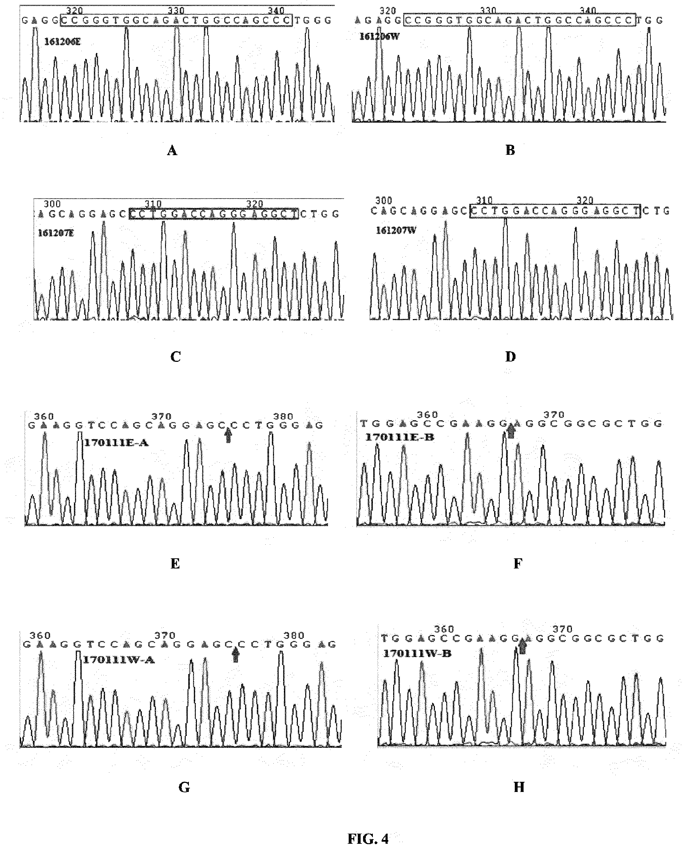

FIG. 4 shows a sequencing peak diagram of APOE gene edited canine. The numbers in the figure are serial number of canine, letter E indicates ear tissue, and letter W indicates tail tissue; 161206E/W indicates ear tissue and tail tissue of wild type canine respectively, and the targeting site region of the wild type gene is indicated in the box (see FIGs.4A and 4B); 161207E/W indicates ear tissue and tail tissue of male canine having APOE gene mutation respectively, and the mutated sequence information is indicated in the box (see FIGs.4C and 4D); 170111E/W indicates ear tissue and tail tissue of female canine having APOE gene mutation respectively, A and B are serial numbers of heterozygous mutation allele, and the arrows point out the mutation region (see FIGs. 4E-4H).

### Embodiment 4: Blood lipid detection of APOE gene knock-out canine

Blood is collected from three-month APOE gene knock-out canine (161207), and is centrifugated for separating serum. The contents of total cholesterol, triglyceride, high-density lipoprotein and low-density lipoprotein in serum were measured. The results show that compared with control canines (numbered 161205 and 161206, respectively), the contents of total cholesterol, triglyceride, high density lipoprotein and low density lipoprotein in serum of APOE gene knock-out canine are apparently higher than the control group (Table 2). It can be seen that the knock-out of APOE gene causes abnormal metabolism of lipids in the gene knock-out canine, leading to significant increase of blood lipid, which further verifies that the present invention obtains the APOE gene knock-out canine.

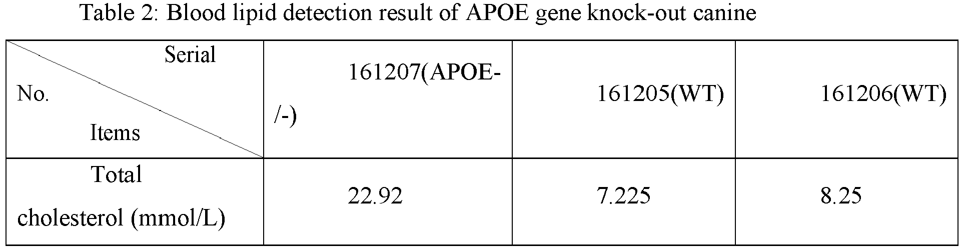

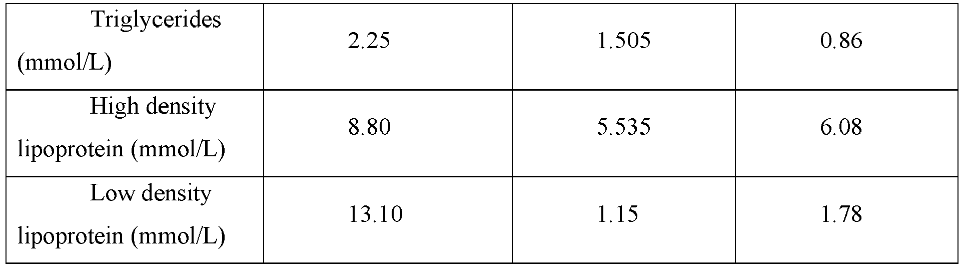

## Claims

1. A method for establishing an APOE gene knock-out canine model demonstrating an atherosclerotic phenotype, comprising the following steps:
(1) determining a targeting site sequence directed to an exon sequence of canine APOE gene sequence; (2) synthesizing sgRNA sequence and the complementary sequence thereof according to the targeting site sequence determined in step (1), then linking the synthesized sequence with a skeleton vector to construct a sgRNA targeting vector; (3) in vitro transcripting the sgRNA targeting vector to obtain mRNA thereof, and in vitro transcripting CRISPR/Cas9 to obtain mRNA thereof; (4) mixing the mRNA of sgRNA and mRNA of CRISPR/Cas9 obtained in step (3), and then intracytoplasmic injecting thereof into the fertilized ovum; and (5) transplanting the fertilized ovum into less bleeding fallopian tubes of a female canine of which both fallopian tubes have been embryo flushed;
the exon in step (1) is exon 3 whose sequence is as shown in SEQ ID NO: 2; and the targeting site sequence is the sequence determined directed to exon 3 as follows: 5'-CCGGGTGGCAGACTGGCCAGCCC-3' (SEQ ID NO: 4); and
the synthesized sgRNA sequence and the complementary sequence thereof in step (2) are as follows:
sgRNA sequence: ataGGGCTGGCCAGTCTGCCACCgt (SEQ ID NO: 5); and
the complementary sequence of sgRNA sequence: taaaacGGTGG CAGACTGGCCAGCC (SEQ ID NO: 6).

2. A canine APOE gene knock-out targeting vector, **characterized in that** the canine APOE gene knock-out targeting vector consists of sgRNA sequence and the complementary sequence thereof designed directed to the targeting site sequence in the exons of canine APOE gene, and the skeleton vector;
the exon is exon 3of the canine APOE gene, the sequence of exon 3 is as shown in SEQ ID NO: 2;
the targeting site sequence is determined as follows: 5'-CCGGGTGGCAGACTGGCCAGCCC-3' (SEQ ID NO: 4); and
the sgRNA sequence and the complementary sequence thereof are as follows:
sgRNA sequence: ataGGGCTGGCCAGTCTGCCACCgt (SEQ ID NO: 5); and
the complementary sequence of sgRNA sequence: taaaacGGTGG CAGACTGGCCAGCC (SEQ ID NO: 6).

3. Somatic cells, tissues and organs of APOE gene knock-out canine obtained by the method of claim 1, wherein the somatic cells comprises a sequence fragment as shown by cctggaccagggaggct (SEQ ID NO: 7).

4. Ear fibroblast BGD-APOEKO-EFO of APOE gene knock-out beagle canine, which is deposited in China General Microbiological Culture Collection Center (CGMCC) on Mar 1st, 2017 with a CGMCC deposit No. 13804.

5. A primer pair for detecting APOE gene knock-out canine comprising a genomic sequence comprising a sequence fragment as shown by cctggaccagggaggct (SEQ ID NO: 7), **characterized in that** the primer pair is designed directed to the sequence as shown by cctggaccagggaggct (SEQ ID NO: 7);
the sequences of the primer pair are as follows:
Forward primer F: 5'-CATTGTTGTCAGGCAGGTAGC-3' (SEQ ID NO: 8); and
Reverse primer R: 5'- GAAGGGTGCGAGGGATTGA-3' (SEQ ID NO: 9).

6. A kit for detecting APOE gene knock-out canine comprising a genomic sequence comprising a sequence fragment as shown by cctggaccagggaggct (SEQ ID NO: 7), **characterized in that** the kit comprises a primer pair designed directed to the sequence as shown by cctggaccagggaggct (SEQ ID NO: 7);
the sequences of the primer pair are as follows:
Forward primer F: 5'-CATTGTTGTCAGGCAGGTAGC-3' (SEQ ID NO: 8); and
Reverse primer R: 5'- GAAGGGTGCGAGGGATTGA-3' (SEQ ID NO: 9).

## Patentansprüche

1. Ein Verfahren zur Erstellung eines APOE-Gen-Knockout-Hundemodells, das einen atherosklerotischen Phänotyp zeigt, umfassend die folgenden Schritte:
(1) Bestimmen einer Targeting-Stellen-Sequenz, die auf eine Exon-Sequenz der Hund-APOE-Gensequenz gerichtet ist; (2) Synthetisieren von sgRNA-Sequenz und der komplementären Sequenz davon gemäß der in Schritt (1) bestimmten Targeting-Stellen-Sequenz, dann Verknüpfen der synthetisierten Sequenz mit einem Skelettvektor, um einen sgRNA-Targeting-Vektor zu konstruieren; (3) In-vitro-Transkription des sgRNA-Targeting-Vektors, um mRNA davon zu erhalten, und In-vitro-Transkription von CRISPR/Cas9, um mRNA davon zu erhalten; (4) Mischen der in Schritt (3) erhaltenen mRNA von sgRNA und mRNA von CRISPR/Cas9, und dann intrazytoplasmatische Injektion davon in die befruchtete Eizelle; und (5) Transplantation der befruchteten Eizelle in weniger blutende Eileiter eines weiblichen Hundes, dessen beide Eileiter embryonal gespült worden sind;
das Exon in Schritt (1) ist Exon 3, dessen Sequenz wie in SEQ ID NO: 2 gezeigt ist; und die Targeting-Stellen-Sequenz ist die bestimmte Sequenz, die wie folgt auf Exon 3 gerichtet ist: 5'-CCGGGTGGCAGACTGGCCAGCCC-3' (SEQ ID NO: 4); und
die synthetisierte sgRNA-Sequenz und die komplementäre Sequenz davon in Schritt (2) sind wie folgt:
sgRNA-Sequenz: ataGGGCTGGCCAGTCTGCCACCgt (SEQ ID NO: 5); und
die komplementäre Sequenz der sgRNA-Sequenz: taaaacGGTGGCAGACTGGCCAGCC (SEQ ID NO: 6).

2. Ein Hunde-APOE-Gen-Knockout-Targeting-Vektor, **dadurch gekennzeichnet, dass** der Hunde-APOE-Gen-Knockout-Targeting-Vektor aus einer sgRNA-Sequenz und der davon entworfenen komplementären Sequenz, die auf die Targeting-Stellen-Sequenz in den Exons des Hunde-APOE-Gens gerichtet ist, und dem Skelettvektor besteht;
das Exon ist Exon 3 des Hunde-APOE-Gens, die Sequenz von Exon 3 ist wie in SEQ ID NO: 2 gezeigt;
die Targeting-Stellen-Sequenz ist wie folgt bestimmt: 5'-CCGGGTGGCAGACTGGCCAGCCC-3' (SEQ ID NO: 4); und
die sgRNA-Sequenz und die komplementäre Sequenz davon sind wie folgt:
sgRNA-Sequenz: ataGGGCTGGCCAGTCTGCCACCgt (SEQ ID NO: 5); und die komplementäre Sequenz der sgRNA-Sequenz: taaaacGGTGGCAGACTGGCCAGCC (SEQ ID NO: 6).

3. Somatische Zellen, Gewebe und Organe von APOE-Gen-Knockout-Hunden, erhalten durch das Verfahren nach Anspruch 1, wobei die somatischen Zellen ein Sequenzfragment, wie durch cctggaccagggaggct (SEQ ID NO: 7) gezeigt, umfassen.

4. Ohr-Fibroblast BGD-APOEKO-EFO von APOE-Gen-Knockout-Beagle-Hunden, der am 1. März 2017 im China General Microbiological Culture Collection Center (CGMCC) mit der CGMCC-Hinterlegungsnummer 13804 hinterlegt wurde.

5. Ein Primer-Paar zum Nachweis von APOE-Gen-Knockout-Hunden umfassend eine genomische Sequenz, die ein Sequenzfragment, wie durch cctggaccagggaggct (SEQ ID NO: 7) gezeigt, umfasst, **dadurch gekennzeichnet, dass** das Primer-Paar auf die Sequenz, wie durch cctggaccagggaggct (SEQ ID NO: 7) gezeigt, gerichtet entworfen ist;
die Sequenzen des Primer-Paares sind wie folgt:
Vorwärts-Primer F: 5'-CATTGTTGTCAGGCAGGTAGC-3' (SEQ ID NO: 8); und
Rückwärts-Primer R: 5'-GAAGGGTGCGAGGGATTGA-3' (SEQ ID NO: 9).

6. Ein Set zum Nachweis von APOE-Gen-Knock-out-Hunden, umfassend eine genomische Sequenz, die ein Sequenzfragment, wie durch cctggaccagggaggct (SEQ ID NO: 7) gezeigt, umfasst, **dadurch gekennzeichnet, dass** das Set ein Primer-Paar umfasst, das gegen die Sequenz, wie durch cctggaccagggaggct (SEQ ID NO: 7) gezeigt, gerichtet entworfen ist;
die Sequenzen des Primer-Paares sind wie folgt:
Vorwärts-Primer F: 5'-CATTGTTGTCAGGCAGGTAGC-3' (SEQ ID NO: 8); und
Rückwärts-Primer R: 5'-GAAGGGTGCGAGGGATTGA-3' (SEQ ID NO: 9).

## Revendications

1. Procédé pour l'établissement d'un modèle canin d'inactivation du gène APOE démontrant un phénotype athérosclérotique, comprenant les étapes suivantes :
(1) détermination d'une séquence de site de ciblage dirigée vers une séquence d'exon d'une séquence du gène APOE canin ; (2) synthèse d'une séquence d'ARNsg et de la séquence complémentaire correspondante selon la séquence de site de ciblage déterminée dans l'étape (1), puis liaison de la séquence synthétisée avec un vecteur squelettique pour construire un vecteur de ciblage d'ARNsg ; (3) transcription *in vitro* du vecteur de ciblage d'ARNsg pour obtenir un ARNm correspondant, et transcription *in vitro* de CRISPR/Cas9 pour obtenir un ARNm correspondant ; (4) mélange de l'ARNm d'ARNsg et de l'ARNm de CRISPR/Cas9 obtenus dans l'étape (3), et ensuite injection intracytoplasmique de ceux-ci dans l'ovule fécondé ; et (5) transplantation de l'ovule fécondé dans des trompes de Fallope saignant moins d'un chien femelle dont l'embryon a été évacué des deux trompes de Fallope ;
l'exon dans l'étape (1) étant l'exon 3 dont la séquence est telle que présentée dans la SEQ ID NO: 2 ; et la séquence de site de ciblage étant la séquence déterminée dirigée vers l'exon 3 comme suit : 5'-CCGGGTGGCAGACTGGCCAGCCC-3' (SEQ ID NO: 4);
et la séquence d'ARNsg synthétisée et la séquence complémentaire correspondante dans l'étape (2) étant comme suit :
séquence d'ARNsg : ataGGGCTGGCCAGTCTGCCACCgt (SEQ ID NO: 5); et
la séquence complémentaire à la séquence d'ARNsg : taaaacOGTGQCAGACTGGCCAGCC (SEQ ID NO: 6).

2. Vecteur ciblant l'inactivation du gène APOE canin, **caractérisé en ce que** le vecteur ciblant l'inactivation du gène APOE canin est constitué d'une séquence d'ARNsg et de la séquence complémentaire correspondante conçue comme étant dirigée vers la séquence de site de ciblage dans les exons du gène APOE canin, et le vecteur squelettique ;
l'exon étant l'exon 3 du gène APOE canin, la séquence de l'exon 3 étant telle que présentée dans la SEQ ID NO: 2 ;
la séquence de site de ciblage étant déterminée comme suit : 5'-CCGGGTGGCAGACTGGCCAGCCC-3' (SEQ ID NO: 4);
et la séquence d'ARNsg et la séquence complémentaire correspondante étant comme suit :
séquence d'ARNsg : ataGGGCTGGCCAGTCTGCCACCgt (SEQ ID NO: 5); et
la séquence complémentaire à la séquence d'ARNsg : taaaacGGTGGCAGACTGGCCAGCC (SEQ ID NO: 6).

3. Cellules, tissus et organes somatiques de canidé à inactivation du gène APOE obtenus par le procédé selon la revendication 1, les cellules somatiques comprenant un fragment de séquence tel que représenté par cctggaccagggaggct (SEQ ID NO: 7).

4. Fibroblaste auriculaire BGD-APOEKO-EFO de canidé de type beagle à inactivation du gène APOE, qui est déposé dans le China General Microbiological Culture Collection Center (CGMCC, centre de collection de cultures microbiologiques général de Chine) le 1^{e} Mars 2017 sous le numéro de dépôt CGMCC n° 13 804.

5. Paire d'amorces pour la détection d'un canidé à inactivation du gène APOE comprenant une séquence génomique comprenant un fragment de séquence tel que représenté par cctggaccagggaggct (SEQ ID NO: 7), **caractérisée en ce que** la paire d'amorces est conçue pour être dirigée vers la séquence telle que représentée par cctggaccagggaggct (SEQ ID NO: 7) ;
les séquences de la paire d'amorces étant comme suit :
amorce avant F : 5'-CATTGTTGTCAGGCAGGTAGC-3' (SEQ ID NO: 8); et
amorce inverse R : 5'- GAAGGGTGCGAGGGATTGA-3' (SEQ ID NO: 9).

6. Kit pour la détection d'un canidé à inactivation du gène APOE comprenant une séquence génomique comprenant un fragment de séquence tel que représenté par cctggaccagggaggct (SEQ ID NO: 7), **caractérisé en ce que** le kit comprend une paire d'amorces conçue pour être dirigée vers la séquence telle que représentée par cctggaccagggaggct (SEQ ID NO: 7);
les séquences de la paire d'amorces étant comme suit :
amorce avant F : 5'-CATTGTTGTCAGGCAGGTAGC-3' (SEQ ID NO: 8); et
amorce inverse R : 5'- GAAGGGTGCGAGGGATTGA-3' (SEQ ID NO; 9).


### SEQUENCE LISTING

<110> Beijing Sinogene Biotechnology Co., Ltd.
<120> Method for preparing a canine model of atherosclerosis
<130> 08-2017-008
<160> 12
<170> PatentIn version 3.5
<210> 1
   <211> 43
   <212> DNA
   <213> Canis lupus
<400> 1
   atgaaggttc tgtgggctgc gctggtggtc acgctcctgg cag 43
<210> 2
   <211> 211
   <212> DNA
   <213> Canis lupus
<400> 2
   gatgctgggc cgatgtgcag ccggagccgg agctggagcg cgagctggag ccgaaggtcc      60
   agcaggagct ggagccagag gccgggtggc agactggcca gccctgggag gcggcgctgg     120
   cccgcttctg ggattacctg cgctgggtgc agacgctgtc tgaccaggtg caagagggcg     180
   tgctcaacac ccaggtcacc caggaactga c                                     211
<210> 3
   <211> 718
   <212> DNA
   <213> Canis lupus
<400> 3
   ggcgctgatg gatgagacca tgaaggaggt gaaggcctac aaggcggagc tggacgagca 60
   gctgggcccc atgacctcgg agacgcaggc ccgcgtggcc aaggagctgc aggcggcgca 120
   ggcccggctg cgctcggaca tggaggacgt gcgcaaccgc ctgacgcagt accgcggcga 180
   gctgcaggcc atgctgggcc agagcagcga ggagctgcgg gcgcgcttcg cctcccacat 240
   gcgcaagctg cgcaagcggg tgctgcggga cgccgaggac ctgcagaggc gcctggccgt 300
   ctacaaggcc ggcgtgcgcg agggtgccga gcgcagcgtg agcagcatcc gcgagcgcct 360
   ctggccgctg ctggagcagg cccgcgagcg caacgccaag gtgggcgccc tggccacgca 420
   gccgctgctc gagcgggccg acgccctggg ccagcagctg cgcgggcagc tggaggagat 480
   gagcagccgg gcccgcggcc acctggagga gatgcgcgag cagatacagg aggtgcgggt 540
   gaagatggag gagcaggccg accagatacg ccaaaaggcc gaggccttcc aggcgcgcct 600
   caagagctgg ttcgagcccc tgctggaaga catgcagcgc cagtgggacg ggctggtgga 660
   gaaggtgcag gcggccgtgg ccaccatccc cacctctaag cctgtggagg aaccatga 718
<210> 4
   <211> 23
   <212> DNA
   <213> Canis lupus
<400> 4
   ccgggtggca gactggccag ccc 23
<210> 5
   <211> 25
   <212> DNA
   <213> Artificial Sequence
<220>
   <223> sgRNA
<400> 5
   atagggctgg ccagtctgcc accgt 25
<210> 6
   <211> 25
   <212> DNA
   <213> Artificial Sequence
<220>
   <223> sequence complementary to sgRNA
<400> 6
   taaaacggtg gcagactggc cagcc 25
<210> 7
   <211> 17
   <212> DNA
   <213> Unknown
<220>
   <223> sequence after mutated sequence
<400> 7
   cctggaccag ggaggct 17
<210> 8
   <211> 21
   <212> DNA
   <213> Artificial Sequence
<220>
   <223> forward primer
<400> 8
   cattgttgtc aggcaggtag c 21
<210> 9
   <211> 19
   <212> DNA
   <213> Artificial Sequence
<220>
   <223> reverse primer
<400> 9
   gaagggtgcg agggattga 19
<210> 10
   <211> 20
   <212> DNA
   <213> Artificial Sequence
<220>
   <223> sgRNA
<400> 10
   gggctggcca gtctgccacc 20
<210> 11
   <211> 194
   <212> DNA
   <213> Unknown
<220>
   <223> mutated sequence
<400> 11
   gatgctgggc cgatgtgcag ccggagccgg agctggagcg cgagctggag ccgaaggtcc        60
   agcaggagcc ctggaccagg gaggctctgg gaggcggcgc tggcccgctt ctgggattac       120
   ctgcgctggg tgcagacgct gtctgaccag gtgcaagagg gcgtgctcaa cacccaggtc       180
   acccaggaac tgac                                                         194
<210> 12
   <211> 17
   <212> DNA
   <213> Unknown
<220>
   <223> sequence prior to mutated sequence
<400> 12
   cctggaccag ggaggct 17